# EUROPÄISCHE PATENTSCHRIFT

(11) **EP 2 623 133 B1**
(45) Veröffentlichungstag und Bekanntmachung des Hinweises auf die Patenterteilung: **02.04.2014**
(21) Anmeldenummer: 12000651.5
(22) Anmeldetag: 01.02.2012
(51) Int. Cl.: A61L 27/26, A61L 27/56, A61L 27/58

(54) **Implant-matrix aus einem Polymergemisch**
Implant matrix from a polymer mixture
Matrice d'implant à base d'un mélange polymère

(43) Veröffentlichungstag der Anmeldung: 07.08.2013
(73) Patentinhaber: Bioenergy Capital AG, 50668 Köln (DE)
(72) Erfinder: Görne, Martin, DE-22337 Hamburg (DE); Kordick, Thomas, DE-63773 Goldbach (DE)
(74) Vertreter: Diehl & Partner GbR

(56) Entgegenhaltungen:
- WO-A1-2006/061229
- WO-A2-00/78928
- US-A- 5 522 895
- US-A1- 2005 042 253

## Beschreibung

Die vorliegende Anmeldung bezieht sich auf poröse Matrices für chirurgische Zwecke.

Zellimplantate auf der Basis poröser Matrices aus bioverträglichen Polymeren sind aus WO 2004/108810 A1 und WO 2006/061229 A1 bekannt. Bei solchen Matrices sind die Poren vernetzt und dienen als Templat für die Ansiedlung von Zellen *in vivo* (z. B. therapeutisch) oder *in vitro* (z. B. diagnostisch). Bei Transplantationen kann eine solche bioresorbierbare Matrix zur temporären Lokalisation des Transplantats und als Platzhalter für sich nach und nach bildendes Gewebe dienen.

Die bekannten Template sind bei einigen Anwendungen noch nicht voll befriedigend, insbesondere hinsichtlich der klinischen Ergebnisse.

Die Erfindung setzt sich daher zum Ziel, eine Verbesserung der klinischen Performance der Template zu erreichen.

Dazu schlägt die Erfindung ein poröses Templat aus einem Gemisch von unterschiedlich schnell abbaubaren Polymeren vor, wobei sich nominelle Resorptionszeiten von zwei der Gemischkomponenten, die jeweils mindestens 10 % des Gemisches ausmachen, um einen Faktor von mindestens 5 unterscheiden. Ohne Beschränkung auf die vermutete Wirkungsweise nehmen die Erfinder an, dass das schneller resorbiert werdende Polymer nach und nach Platz schafft für die sich bildenden Gefäße, während der Zusammenhalt der Gesamtstruktur durch das langsamer resorbiert werdende Polymer gewährleistet ist, ohne dass einzelne Strukturelemente als Fremdkörper wirken. Zudem verändert der fortschreitende Abbau des schneller resorbiert werdenden Polymers das physiologische Milieu in für den therapeutischen Erfolg günstiger Weise.

Unter einem weiteren Aspekt schlägt die Erfindung Verfahren zur Herstellung von porösen bioresorbierbaren Matrices vor, wobei ein Gemisch aus mindestens zwei unterschiedlich schnell resorbierbaren Polymeren und einem wasserlöslichen Porenbildner und einem Lösungsmittel für eines der Polymere hergestellt, gefolgt von Abdampfen des Lösungsmittels und Wässern zur Porenbildung. In Varianten wird das Gemisch nach dem Abdampfen des Lösungsmittels kompaktiert. Beide Verfahren führen zu hochporösen Polymermatrixscheiben, deren klinische Performance hervorragend ist. Die Abbauzeiten der Polymere unterscheiden sich um einen Faktor 5 oder mehr.

Erfindungsgemäß ist die poröse Matrix hydrophil mit Acrylsäure-Polymerisat beschichtet. Dazu wird ein Plasma-Beschichtungsschritt gefolgt von einem plasmalosen Beschichtungsschritt, wodurch die benötigten Schichtdicken von über einem Mikrometer erreicht werden. Die initial aufgebrachte Teil-Schicht ist dünner als die plasmalos aufgebrachte Teil-Schicht. Die Beschichtung führt zu einer weiteren Verbesserung der Zellanhaftung.

Weitere Merkmale der Erfindung ergeben sich aus der nachfolgenden Beschreibung von Ausführungsbeispielen in Verbindung mit den Ansprüchen. Die Erfindung ist nicht auf die beschriebenen Ausführungsbeispiele beschränkt, sondern durch den Umfang der beiliegenden Patentansprüche bestimmt. Insbesondere können die einzelnen Merkmale bei erfindungsgemäßen Ausführungsformen in anderer Anzahl und Kombination als bei den untenstehend angeführten Beispielen verwirklicht sein.

In einer Hauptanwendung werden Matrices zur Defektdeckung bereitgestellt, beispielsweise einer Hernien-Dehiszenz. Es ist vorgesehen, dass ein erster Teil der verwendeten Polymermischung schneller abgebaut wird und ein anderer Teil der Polymermischung langsamer erodiert (Verhältnis der Abbauzeiten mindestens 5) und den strukturellen Zusammenhalt längere Zeit, z. B. 2,5-3 Jahre (oder mindestens 2 und/oder weniger als 5 Jahre) gewährleistet. Durch die allmähliche Auflösung des schneller abbaubaren Teils der Matrix binnen 3-4 Monaten, oder mindestens 2 und/oder weniger als 7 Monaten wird das physiologische Milieu in einer Weise beeinflusst, die dem therapeutischen Erfolg dienlich ist. Solche Polymere sind zweckmäßig auf Basis von α-Hydroxycarbonsäuren wie Milchsäure und/oder Glykolsäure, z. B. PLA oder PLGA. Die Hersteller solcher zum Einsatz im menschlichen Körper zugelassener Polymere geben die hier relevanten nominellen Abbauzeiten an. Die hier verwendeten Polymere sind z. B. von der Fa. Evonik erhältlich und tragen die Bezeichnungen L210s, L210, L09s, L207s, L206s (langsamer abbaubare PLGA-Polymere) bzw. RG502, RG502H, RG505 (schneller abbaubare PLGA-Polymere).

In der Hauptvariante werden die erfindungsgemäßen Matrices mechanisch hinreichend stabil hergestellt, dass sie z. B. den Belastungen durch chirurgische Nähvorgänge standhalten. An ihrer Peripherie können die Matrices dadurch mit Körpergewebe verbunden werden. Ihre Porosität stellt sicher, dass die Matrices mit Bindegewebszellen infiltriert werden. Eine besonders gute Anhaftung wird durch eine Beschichtung mit PAA in einem kombinierten PECVD/CVD-Prozess erreicht, bei der eine initiale Plasma-erzeugte Lage als Haftvermittler für eine nachfolgende kristalline PAA-Schicht dient. Gemäß einer Ausführungsform weist die Matrix eine porenarme oder -frei Seite auf, die für die eigentliche Abdeckung sorgt, und eine porenreiche, die für die Infiltration günstig ist. Im Körper kann dabei die glattere porenarme Seite dem Körperinneren zugewandt angeordnet werden, um bei Ausübung von Druck durch die Körperorgane auf die Defektstelle keine Angriffsstelle zu bieten.

In einer Variante wird die Matrix vorab beispielsweise mit Hepatozyten und/oder mit Langerhans'schen Inselzellen infiltriert. Solche biochemischen Funktionszellen heften sich an die Innenwände der Poren der schaumartigen Matrix an (Anheftungsraten über 80 % oder, geeignet beschichtet, über 95 %) und können mit der Matrix in mesotheliale Taschen transplantiert werden, idealerweise des Zellspenders selbst. Dabei wird ausgenutzt, dass in diesem Fall keine Abstoßungsreaktion erfolgt, sondern nur ein vergleichsweise milder, für den therapeutischen Prozess günstiger Fremdkörper-Reiz ausgeübt wird. Binnen weniger Wochen wird die Matrix vaskularisiert und sind die implantierten Zellen nicht mehr nur auf diffusive Versorgung angewiesen. Die Matrices werden so angeordnet, dass die porenarme (oder -freie) Seite innen und die porenreiche Seite außen liegt, um die Verlustrate durch Abwanderung der Zellen niedrig zu halten.

Wie bereits erwähnt, werden besonders gute Anheftungsraten bei beschichteten Matrices beobachtet, nämlich solchen, die in einem kombinierten PECVD/CVD-Verfahren zunächst mit einer dünnen (z. B. 20-30 nm) PAA-Lage plasmabeschichtet und daran anschließend mit einer dickeren PAA-Lage (z. B. 20-30 µm) ohne Plasma-Einwirkung beschichtet werden. Diese obere Lage bildet eine kristalline, hydrophile Schicht aus.

Zunächst wird in einer Ausführungsform eine Lösung eines der verwendeten Polymere in für medizinische Zwecke zugelassenem Chloroform in eine Form gegossen und das Lösungsmittel bei 45°-65° abgedampft. Daraufhin wird eine Polymer-Mischung definierter Partikelgrößen-Verteilung mit einem Kochsalz-Granulat ebenfalls definierter Partikelgrößen-Verteilung gemischt, mit einer Lösung eines der Polymere in Chloroform vermengt und dann auf die bereits hergestellte Polymerschicht gegeben. Aus diesem Vorformling dampft das Lösungsmittel bei leicht erhöhter Temperatur (45-65°C) ab; sodann kann er gewünschtenfalls durch Beaufschlagung mit Druck kompaktiert werden. Anschließend wird der Kompaktkörper gewässert, um durch Entfernen des Salzes die gewünschte Porosität bereitzustellen. Dabei bleibt eine initial hergestellte Polymerschicht porenfrei. Je nach Einsatzzweck kann die Dicke der porenarmen Schicht durch Menge und Konzentration der anfänglichen Lösung eingestellt werden. Beispielsweise erhält man eine stabile Membran, wenn bei einer Konzentration der Lösung von z. B. 4 % in Chloroform (langsam abbaubares Polymer) die Füllhöhe etwa 5-50 mm, typisch 20-25 mm beträgt. Das Abdampfen des Chloroforms dauert dann etwa 1,5 h und resultiert in einer Schichtdicke von ca. 0,5-2 mm. Im anderen Fall geht man bei gleicher Polymer-Konzentration von einer Füllhöhe von nur 0,1-0,5 mm aus, wodurch das Abdampfen des Chloroforms schneller beendet ist (ca. 20-30 min), und die entstehende Membran eine Dicke von nur ca. 10-20 µm hat.

Die Kochsalzpartikel der porenbildenden Mischung sind etwas gröber (Median bei 400-420 µm) als die Polymerpartikel (Median des langsamer abbaubaren Polymers zwischen 210 µm und 230 µm, der des schneller abbaubaren Polymers zwischen 150 µm und 170 µm). Dabei sind die Verteilungsbreiten (5 %/95 %) ähnlich, nämlich etwa ± 85-95 µm für Salz bzw. Polymer insgesamt. Die Verteilungsform kann bi- oder trimodal sein. Die Zusammensetzung der Schichtmischung ist zu etwa 96 % Salz, 1-1,5 % festes Polymer und weitere ca. 3-5 % gelöstes Polymer, wobei die Volumenanteile von Feststoffen und Flüssigkeit etwa gleich sind. Insgesamt beträgt der Anteil des schnell abbaubaren Polymers lediglich etwa 5-20 % des Polymers. Die Gesamtdicke der porenbildenden Schicht ist 4-5 mm. In der Variante einer fragileren Initialschicht kann das Salz etwas feiner gewählt werden (Median ca. 350-370 µm). In diesem Fall ist die Gesamtdicke der porenbildenden Schicht 5-6 mm. Das Wässern dauert ca. 24 h und wird gefolgt von einer Trocknung bei 45-50°C. Wenn eine Beschichtung vorgenommen wird, liegt die Matrix mit der porenarmen Seite (wenn vorhanden) auf und wird daher überwiegend die offenporige Seite beschichtet.

Bei einer Verwendung außerhalb des Körpers kann eine Matrix gemäß der obigen Beschreibung zur Fixierung von Zellen dienen, die in einem Bioreaktor Agenzien ausgesetzt werden. Beispielsweise können definierte Zelltypen auf diese Weise daraufhin untersucht werden, ob sie auf in Frage kommende Medikamente ansprechen oder nicht, und kann die Therapie in Abhängigkeit von derart erhaltenen Untersuchungsergebnissen geplant werden. Ebenso kann die Medikamentenentwicklung vereinfacht werden, weil Toxizität frühzeitig erkannt wird.

Der Fachmann wird erkennen, dass im Rahmen des Schutzumfangs der beigefügten Ansprüche Abwandlungen der oben beschriebenen Beispiele möglich sind.

## Patentansprüche

1. Poröse Implantat-Matrix, die überwiegend aus einem Gemisch von unterschiedlich schnell abbaubaren Polymeren besteht, wobei sich nominelle Resorptionszeiten von zwei der Gemischkomponenten, die jeweils mindestens 10 % des Gemisches ausmachen, um einen Faktor von mindestens 5 unterscheiden, wobei die Matrix eine mit Polyacrylsäure, PAA, hydrophilierend beschichtete Oberfläche aufweist, wobei eine initial unter Einwirkung eines Plasmas aufgebrachte PAA-Schichtdicke geringer ist als eine danach ohne Plasma-Einwirkung aufgebrachte PAA-Schichtdicke.

2. Poröse Implantat-Matrix nach Anspruch 1, wobei die Gemischkomponenten Poly(α-hydroxy)carbonsäuren sind.

3. Poröse Implantat-Matrix nach Anspruch 2, wobei die Gemischkomponenten PLA und PLGA sind.

4. Poröse Implantat-Matrix nach einem der vorstehenden Ansprüche, wobei die hydrophilierte Oberfläche Acrylsäureeinheiten aufweist.

5. Poröse Implantat-Matrix nach einem der vorstehenden Ansprüche, wobei die Matrix eine Porosität von mindestens 80 % aufweist.

6. Poröse Implantat-Matrix nach einem der vorstehenden Ansprüche, wobei eine nominelle Resorptionszeit des schneller abbaubaren Polymers weniger als 4 Monate beträgt.

7. Poröse Implantat-Matrix nach einem der vorstehenden Ansprüche, wobei eine nominelle Resorptionszeit des langsamer abbaubaren Polymers mehr als 20 Monate beträgt.

8. Verwendung der porösen Implantat-Matrix nach einem der vorstehenden Ansprüche zum Besiedeln mit vitalen Zellen außerhalb des Körpers.

9. Verwendung nach Anspruch 8, ferner umfassend Aussetzen der Zellen einem vorbestimmten Test-Agens.

10. Verfahren zur Herstellung einer porösen Implantat-Matrix aus einem Gemisch von mindestens zwei unterschiedlich abbaubaren Polymeren, wobei Partikel der beiden Polymere mit Partikeln eines wasserlöslichen Feststoffs und einer Lösung wenigstens eines der Polymere in einem mit Wasser nicht mischbaren Lösungsmittel gemischt werden, und der Feststoff dann durch Wässern entfernt wird, wobei sich nominelle Resorptionszeiten der Gemischkomponenten, die jeweils mindestens 10 % des Gemisches ausmachen, um einen Faktor von mindestens 5 unterscheiden, ferner umfassend Beschichten mit einem hydrophilierenden Material nach dem Wässern, wobei einem PAA-Plasma-beschichtungsschritt ein PAA-Beschichtungsschritt ohne Plasma derart folgt, dass die initial unter Einwirkung des Plasmas aufgebrachte PAA-Schichtdicke geringer ist als die danach ohne Plasma-Einwirkung aufgebrachte PAA-Schichtdicke.

11. Verfahren nach Anspruch 10, umfassend Beaufschlagen mit Druck zum Kompaktieren des Gemischs nach dem Abdampfen des Lösungsmittels und vor dem Wässern.

12. Verfahren nach Anspruch 10 oder 11, wobei als Lösungsmittel Chloroform verwendet wird.

13. Verfahren nach einem der Ansprüche 10 bis 12, wobei ein Vorläufer des hydrophilierenden Materials Acrylsäure oder Acrylsäureanhydrid ist.

14. Verfahren nach einem der Ansprüche 10 bis 13, ferner umfassend Besiedeln der Matrix mit vitalen Zellen außerhalb des Körpers und Aussetzen der Zellen einem vorbestimmten Test-Agens.

## Claims

1. A porous implant matrix, mainly consisting of a mixture of differently rapidly resorbable polymers, wherein nominal resorption rates of two of the mixture components, each accounting for at least 10 % of the mixture, differ by a factor of at least 5, wherein the matrix comprises a surface hydrophilizingly coated with poly(acrylic acid), PAA, wherein a PAA layer thickness initially applied under the influence of a plasma is less than a PAA layer thickness subsequently applied without action of the plasma.

2. The porous implant matrix of claim 1, wherein the mixture components are poly((α-hydroxy)carboxylic acids).

3. The porous implant matrix of claim 2, wherein the mixture components are PLA and PLGA.

4. The porous implant matrix of one of the preceding claims, wherein the hydrophilizing surface comprises acrylic acid units.

5. The porous implant matrix of one of the preceding claims, wherein the matrix has a porosity of at least 80 %.

6. The porous implant matrix of one of the preceding claims, wherein a nominal resorption time of the more rapidly resorbable polymer is less than 4 months.

7. The porous implant matrix of one of the preceding claims, wherein a nominal resorption time of the more slowly resorbable polymer is more than 20 months.

8. Use of the porous implant matrix of one of the preceding claims for infiltration with viable cells.

9. The use of claim 8, further comprising exposing the cells to a predetermined test agent.

10. A process for manufacturing a porous implant matrix from a mixture of at least two differently resorbable polymers, wherein particles of both polymers are mixed with particles of a water soluble solid and a solution of at least one of the polymers in a non-water-miscible solvent, and the solid is then removed by watering, wherein nominal resorption times of the mixture components, each accounting for at least 10 % of the mixture, differ by a factor of at least 5, further comprising coating with a hydrophilizing material after the watering, wherein a PAA-plasma coating step is followed by a PAA-coating step without plasma, such that the PAA layer thickness applied initially under the influence of the plasma is less than the PAA layer thickness applied without the action of the plasma.

11. The process of claim 10, comprising applying pressure for compacting the mixture after evaporating the solvent and before the watering.

12. The process of claim 10 or 11, wherein chloroform is used as the solvent.

13. The process of one of claims 10 to 12, wherein a precursor of the hydrophilizing material is acrylic acid or acrylic acid anhydride.

14. The process of one of claims 10 to 13, further comprising infiltrating the matrix with viable cells outside of the body, and exposing the cells to a predetermined test agent.

## Revendications

1. Matrice d'implant poreuse, qui consiste principalement en un mélange de polymères différents rapidement dégradables, dans laquelle des temps de résorption nominaux de deux des composants du mélange qui constituent respectivement 10 % du mélange, se distinguent d'un facteur d'au moins 5, la matrice présentant une surface revêtue de façon à être hydrophile, avec de l'acide polyacrylique, PAA, dans laquelle une épaisseur de couche de PAA appliquée initialement sous l'effet d'un plasma est inférieure à une épaisseur de couche de PAA appliquée ensuite sans l'effet d'un plasma.

2. Matrice d'implant poreuse selon la revendication 1, dans laquelle les composants du mélange sont des acides poly(α-hydroxy)carboxyliques.

3. Matrice d'implant poreuse selon la revendication 2, dans laquelle les composants du mélange sont le PLA et le PLGA.

4. Matrice d'implant poreuse selon l'une des revendications précédentes, dans laquelle la surface rendue hydrophile présente des motifs d'acide acrylique.

5. Matrice d'implant poreuse selon l'une des revendications précédentes, dans laquelle la matrice présente une porosité d'au moins 80 %.

6. Matrice d'implant poreuse selon l'une des revendications précédentes, dans laquelle un temps de résorption nominal du polymère dégradable plus rapidement est inférieur à 4 mois.

7. Matrice d'implant poreuse selon l'une des revendications précédentes, dans laquelle un temps de résorption nominal du polymère dégradable plus lentement est supérieur à 20 mois.

8. Utilisation de la matrice d'implant poreuse selon l'une des revendications précédentes, pour la colonisation avec des cellules vitales, en dehors du corps.

9. Utilisation selon la revendication 8, comprenant en outre l'exposition des cellules à un agent de test prédéterminé.

10. Procédé de production d'une matrice d'implant poreuse à partir d'un mélange d'au moins deux polymères dégradables de façon différente, dans lequel des particules des deux polymères sont mélangées à des particules d'un solide soluble dans l'eau et à une solution d'au moins l'un des polymères dans un solvant non miscible dans l'eau, et le solide est ensuite éliminé par lavage, des temps de résorption nominaux des composants de mélange qui constituent respectivement au moins 10 % du mélange se distinguant d'un facteur d'au moins 5, comprenant en outre des revêtements avec un matériau hydrophilisant, après le lavage, dans lequel une étape de revêtement de PAA au plasma suit une étape de revêtement de PAA sans plasma de telle sorte que l'épaisseur de couche de PAA appliquée initialement sous l'effet du plasma soit inférieure à l'épaisseur de couche de PAA appliquée ensuite sans l'effet du plasma.

11. Procédé selon la revendication 10, comprenant l'application de pression pour compacter le mélange après l'évaporation du solvant et avant le lavage.

12. Procédé selon la revendication 10 ou 11, dans lequel on utilise comme solvant, du chloroforme.

13. Procédé selon l'une des revendications 10 à 12, dans lequel un précurseur du matériau hydrophilisant est un acide acrylique ou un anhydride d'acide acrylique.

14. Procédé selon l'une des revendications 10 à 13, comprenant en outre la colonisation de la matrice avec des cellules vitales en dehors du corps et l'exposition des cellules à un agent de test prédéterminé.
